(19) Europäisches Patentamt
European Patent Office
Office européen des brevets

(11) Veröffentlichungsnummer : **0 494 048 A1**

(12) # EUROPÄISCHE PATENTANMELDUNG

(21) Anmeldenummer : **91810982.8**

(22) Anmeldetag : **16.12.91**

(51) Int. Cl.[5] : **C07D 335/04,** G03F 7/00, G11B 7/24

(30) Priorität : **03.01.91 CH 2/91**

(43) Veröffentlichungstag der Anmeldung : **08.07.92 Patentblatt 92/28**

(84) Benannte Vertragsstaaten : **BE CH DE FR GB IT LI NL SE**

(71) Anmelder : **CIBA-GEIGY AG**
**Klybeckstrasse 141**
**CH-4002 Basel (CH)**

(72) Erfinder : **Fischer, Walter, Dr.**
**Vogesenstrasse 77**
**CH-4153 Reinach (CH)**
Erfinder : **Finter, Jürgen, Dr.**
**Zasiusstrasse 100**
**W-7800 Freiburg (DE)**
Erfinder : **Spahni, Heinz**
**Eggstrasse 23**
**CH-4402 Frenkendorf (CH)**

(54) **Photochrome Benzothioxanthonoxide, Verfahren zu deren Herstellung und deren Verwendung.**

(57) Verbindungen der Formeln I oder III oder Mischungen solcher Verbindungen,

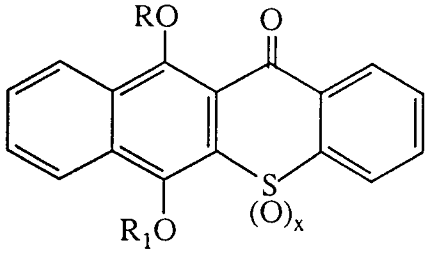

(I),

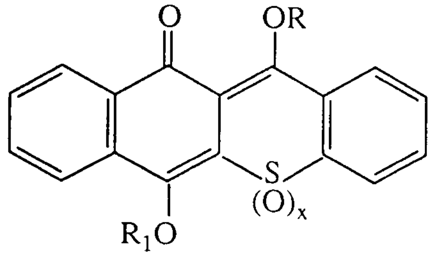

(III),

worin
R unsubstituienes oder mit $C_1$-$C_{12}$-Alkyl, $C_1$-$C_{12}$-Alkoxy, $C_1$-$C_{12}$-Alkylthio, Phenyl, Benzyl, -CN, -$CF_3$, Halogen oder -$COOR_3$ substituiertes $C_6$-$C_{14}$-Aryl bedeutet, x für 1 oder 2 steht und $R_1$ H, lineares oder verzweigtes $C_1$-$C_{12}$-Alkyl, $C_2$-$C_{12}$-Alkenyl oder -Alkinyl, $C_7$-$C_{16}$-Aralkyl, $C_8$-$C_{16}$-Alkaralkyl, -$CH_2COOR_3$ oder $C_1$-$C_{12}$-Acyl darstellt, worin $R_3$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht, sind reversible photochrome Systeme, die zur Kontrastbildung, Lichtabsorption oder zur reversiblen optischen Speicherung von Informationen verwendet werden können.



Jouve, 18, rue Saint-Denis, 75001 PARIS

Die vorliegende Erfindung betrifft in 8-Stellung mit Aryloxygruppen und in 5-Stellung mit einer gegebenenfalls derivatisierten Hydroxylgruppe substituierte 6,7-Benzothioxanthonoxide, ein Verfahren zu deren Herstellung und deren Verwendung als photochrome Systeme zur Kontrastbildung, zur Lichtabsorption und zur Aufzeichnung von Informationen, sowie neue Benzothioxanthonoxide.

Yu. E. Gerasimenko et al. beschreiben im Zhumal Organicheskoi Khimii, Vol. 7, No. 11, S. 2413-2415 (1971) 6-Phenoxy-naphthacen-5,12-dion als reversible photochrome Verbindung, die bei Bestrahlung das orange 5-Phenoxynaphthacen-6, 12-dion (Anachinon) bildet. Yu. E. Gerasimenko et al. beschreiben im Zhumal Organicheskoi Khimii, Vol. 16, No. 9, S. 1938-1945 (1980) 6,11-Diphenoxy-naphthacen-5,12-dion, dessen Photoisomerisation zur Synthese von 6-Aminoderivaten des 12-Phenoxy-naphthacen-5,11-dions verwendet wird.

Ein Gegenstand der Erfindung sind Verbindungen der Formel I oder Mischungen solcher Verbindungen,

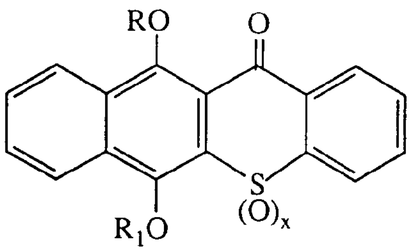

(I),

worin

R unsubstituiertes oder mit $C_1$-$C_{12}$-Alkyl, $C_1$-$C_{12}$-Alkoxy, $C_1$-$C_{12}$-Alkylthio, Phenyl, Benzyl, -CN, $-CF_3$, Halogen oder $-COOR_3$ substituiertes $C_6$-$C_{14}$-Aryl bedeutet, x für 1 oder 2 steht und $R_1$ H, lineares oder verzweigtes $C_1$-$C_{12}$-Alkyl, $C_2$-$C_{12}$-Alkenyl oder -Alkinyl, $C_7$-$C_{16}$-Aralkyl, $C_8$-$C_{16}$-Alkaralkyl, $-CH_2COOR_3$ oder $C_1$-$C_{12}$-Acyl darstellt, worin $R_3$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht.

R in Formel I ist bevorzugt unsubstituiertes oder substituiertes $C_6$-$C_{10}$-Aryl, zum Beispiel Phenyl, 1- oder 2-Naphthyl. Bevorzugt stellt R unsubstituiertes oder substituiertes Phenyl dar.

Die Gruppe R kann mit einem oder mehreren, bevorzugt 1 bis 3 Resten substituiert sein. Wenn R mit Alkyl, Alkoxy oder Alkylthio substituiert ist, können diese linear oder verzweigt sein und bevorzugt 1 bis 6, besonders 1 bis 4 C-Atome enthalten. Beispiele sind Methyl, Ethyl, die Isomeren von Propyl, Butyl, Pentyl, Hexyl, Heptyl, Octyl, Nonyl, Decyl, und die entsprechenden Alkoxy- und Alkylthioreste. Bevorzugt sind Methyl, Ethyl, n-und i-Propyl, n-, i- und t-Butyl, Methoxy, Ethoxy, Methylthio und Ethylthio.

Wenn R mit Halogen substituiert ist, handelt es sich bevorzugt um -Br, -Cl und -F und besonders bevorzugt um -Cl.

$R_3$ in der Bedeutung von Alkyl kann linear oder verzweigt sein. Weitere Beispiele zu den zuvorgenannten Alkylresten sind die Isomeren von Tridecyl, Tetradecyl, Pentadecyl, Hexadecyl, Heptadecyl und Octadecyl. Bevorzugt enthält $R_3$ als Alkyl 1 bis 12, besonders 1 bis 6 C-Atome. $R_3$ als Alkylphenyl ist bevorzugt $C_1$-$C_6$-, besonders $C_1$-$C_4$-Alkylphenyl, z.B. Dodecylphenyl, Octylphenyl, Hexylphenyl, n-, i- oder t-Butylphenyl, n- oder i-Propylphenyl, Ethylphenyl, Methylphenyl. $R_3$ als Alkylbenzyl ist bevorzugt $C_1$-$C_6$-, besonders $C_1$-$C_4$-Alkylbenzyl, z.B. Dodecylbenzyl, Octylbenzyl, Hexylbenzyl, n-, i- oder t-Butylphenyl, n- oder i-Propylbenzyl, Ethylbenzyl, Methylbenzyl. $R_3$ ist bevorzugt H oder $C_1$-$C_{18}$-Alkyl, besonders $C_1$-$C_{12}$-Alkyl.

In einer bevorzugten Ausführungsform ist R in Formel I unsubstituiert oder mit $C_1$-$C_4$-Alkyl, $C_1$-$C_4$-Alkoxy, $C_1$-$C_4$-Alkylthio, -F, -Cl, -Br oder $-COOR_3$ substituiert, wobei $R_3$ H oder $C_1$-$C_{18}$-Alkyl bedeutet.

In einer ganz besonders bevorzugten Ausführungsform stellt R in Formel I Phenyl dar, das unsubstituiert oder mit -Cl, $-COOCH_3$ oder $-COOC_2H_5$ substituiert ist. Beispiele sind Phenyl, p-Chlorphenyl, 3,5-Dichlorphen-1-yl, p-(Carbomethoxy)phenyl und p-(Carboethoxy)-phenyl.

$R_1$ enthält als Alkyl bevorzugt 1 bis 8 C-Atome und besonders bevorzugt 1 bis 6 C-Atome. Bevorzugt handelt es sich um lineares Alkyl. Beispiele sind insbesondere Methyl, Ethyl, n-Propyl, n-Butyl, n-Pentyl und n-Hexyl.

$R_1$ enthält als Alkenyl bevorzugt 2 bis 6 C-Atome. Bevorzugt handelt es sich um lineares Alkenyl. Als Beispiel ist besonders Allyl zu nennen.

$R_1$ enthält als Alkinyl bevorzugt 2 bis 6 C-Atome. Bevorzugt handelt es sich um lineares Alkinyl. Als Beispiel ist besonders Propargyl zu nennen.

$R_1$ enthält als Aralkyl bevorzugt 7 bis 12 C-Atome. Bevorzugt handelt es sich um Phenylalkyl mit 1 bis 4 C-Atomen im Alkylrest. Beispiele sind Phenylbutyl, Phenylpropyl, Phenylethyl und besonders Benzyl.

$R_1$ enthält als Alkaralkyl bevorzugt 8 bis 12 C-Atome. Bevorzugt handelt es sich um ($C_1$-$C_4$-Alkyl)benzyl, zum Beispiel Ethylbenzyl und Methylbenzyl.

In einer bevorzugten Ausführungsform steht $R_1$ in Formel I für $C_1$-$C_6$-Alkyl, Benzyl oder ($C_1$-$C_4$-Alkyl)benzyl.

In den Verbindungen der Formel I steht x bevorzugt für 2.

In einer bevorzugten Ausführungsform steht in den Verbindungen der Formel I R für Phenyl, $R_1$ für H, $C_1$-$C_6$-Alkyl, besonders bevorzugt $C_1$-$C_4$-Alkyl, oder Benzyl, und x für 1 oder 2.

In einer besonders bevorzugten Ausführungsform steht in den Verbindungen der Formel I R für Phenyl, $R_1$ für Methyl, Butyl oder Benzyl, und x für 2.

Ein weiterer Gegenstand der Erfindung ist ein Verfahren zur Herstellung von Verbindungen der Formel I, das dadurch gekennzeichnet ist, dass man eine Verbindung der Formel II

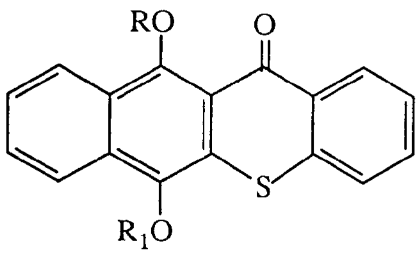

(II),

worin R und $R_1$ die zuvor angegebenen Bedeutungen haben, mit einem Sauerstoff abgebenden Oxidationsmittel umsetzt.

Geeignete Oxidationsmittel sind zum Beispiel Sauerstoff, gegebenenfalls zusammen mit einer Metallverbindung als Katalysator, sowie anorganische und organische Perverbindungen wie zum Beispiel Persäuren oder deren Salze, und Peroxide. Ein bevorzugtes Oxidationsmittel ist Wasserstoffperoxid. Die Reaktion wird vorteilhaft in einem inerten Lösungsmittel durchgeführt, zum Beispiel Halogenkohlenwasserstoffen (Methylenchlorid, Chloroform, Tetrachlorkohlenstoff, 1,1,2,2-Tetrachlorethan, Mono-oder Dichlorbenzol), Sulfonen (Tetramethylensulfon, Dimethylsulfon) sowie Carbonsäureamiden (Dimethylformamid). Bei der Verwendung von Wasserstoffperoxid wird zweckmässig eine organische Säure, zum Beispiel Eisessig, oder eine wässrige Säure als Lösungsmittel verwendet, zum Beispiel Essigsäure.

Die Bildung von Mono- beziehungsweise Dioxiden kann bei der Verwendung von Wasserstoffperoxid im allgemeinen durch die Wahl der Temperatur unter sonst gleichen Reaktionsbedingungen gesteuert werden. Bei Temperaturen bis zu etwa 30 °C werden überwiegend die Monooxide (x in Formel I gleich 1) und bei Temperaturen über etwa 50 °C werden überwiegend die Dioxide (x in Formel I gleich 2) gebildet.

Ein weiterer Gegenstand der Erfindung sind die Verbindungen der Formel II

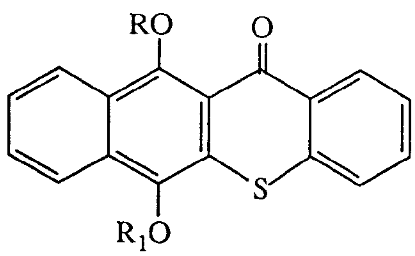

(II),

worin R und $R_1$ die zuvor angegebenen Bedeutungen haben. Die Verbindungen der Formel II weisen in Lösung bei Bestrahlung nur einen irreversiblen Farbumschlag nach schwach grün auf.

Die Verbindungen der Formel II können folgendermassen hergestellt werden: Die Umsetzung des bekannten 1,4-Dihydroxy-3-(2′-carboxyl- 1′-phenylthio)naphthalins mit einem Alkylierungsmittel, zum Beispiel einem Alkyliodid (Methyl- oder Ethyliodid) in Gegenwart einer Metallbase wie zum Beispiel Kaliumcarbonat ergibt 1,4-Dialkoxy-3-(2′-carbalkoxy- 1′-phenylthio)naphthalin. Die Verseifung dieser Verbindung mit zum Beispiel einem Alkalimetallhydroxid in alkoholischer Lösung führt zur entsprechenden Säure, die mit Phosphoroxytrichlorid oder -bromid zu einem Benzothioxanthon der Formel A

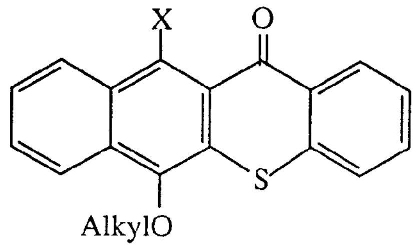

(A),

worin X -Cl oder -Br darstellt, umgesetzt wird.

Die Verbindung A kann direkt mit einem Phenol der Formel ROH gegebenenfalls in Gegenwart einer Alkalimetallbase, einem Alkalimetallcarbonat oder einem tertiären Amin zu Verbindungen der Formel II, worin $R_1$ Alkyl bedeutet, umgesetzt werden. Es ist aber auch möglich, in den Verbindungen der Formel A die Gruppe AlkylO- in eine OH-Gruppe umzuwandeln, zum Beispiel mit einem Halogenwasserstoff, um danach die OH-Gruppe mit einer Verbindung $R_1Y$ zu derivatisieren, wobei Y für eine Abgangsgruppe, zum Beispiel Halogen steht und $R_1$ mit Ausnahme von H und Alkyl die zuvor angegebenen Bedeutungen hat, und erst dann die Umsetzung mit Phenol durchführt.

Verbindungen der Formel I, in denen $R_1$ Methyl bedeutet, können auf die zuvor beschriebene Weise zu anderen Verbindungen der Formel I umgewandelt werden: Durch die Abspaltung der Methylgruppe erhält man die Hydroxylverbindungen der Formel I mit $R_1$ gleich H, die mit einer Verbindung der Formel $R_1Y$ derivatisiert werden können ($R_1$ hat mit Ausnahme von Methyl die zuvor angegebenen Bedeutungen). Die Hydroxylverbindungen der Formel I können durch Umsetzung mit $BBr_3$ in Methylenchlorid in die Verbindungen der Formel B

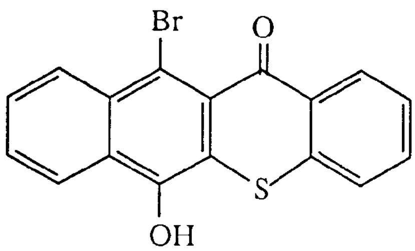

(B)

umgewandelt werden, deren Hydroxylgruppe mit einer Verbindung der Formel $R_1Y$ derivatisiert und dann mit einem Phenol der Formel ROH zu einer Verbindung der Formel I übergeführt werden. Weitere Einzelheiten zu diesen Reaktionen können den Beispielen entnommen werden.

Die Verbindungen der Formel I sind Kristallin, thermisch stabil und farblos bis leicht gelb gefärbt. Sie sind in organischen Lösungsmitteln löslich. Sie sind wirksame Photoinitiatoren und Photosensibilisatoren für photopolymerisierbare Systeme, die ethylenisch ungesättigte Doppelbindungen enthalten. Ferner sind die Verbindungen der Formel I reversibel photochrom.

Bei Bestrahlung der erfindungsgemässen Verbindungen, gegebenenfalls in einem Substrat, mit Licht einer Wellenlange von etwa 300 bis 450 nm wird eine ausgeprägte Farbanderung nach gelb beobachtet. Die Lichtabsorption ist im Vergleich zu 6,11-Diphenoxynaphthacen-5,12-dion zu niedrigerer Wellenlänge verschoben. Die Farbänderung beruht auf der photochemischen Umwandlung der erfindungsgemässen Verbindungen der Formel I in die Verbindungen der Formel III. Die Umwandlungsgeschwindigkeit ist überraschend hoch und kann je nach Menge, Dicke der Probe und Strahlungsintensität unter 3 Sekunden liegen.

Ein weiterer Gegenstand der Erfindung sind die Verbindungen der Formel III

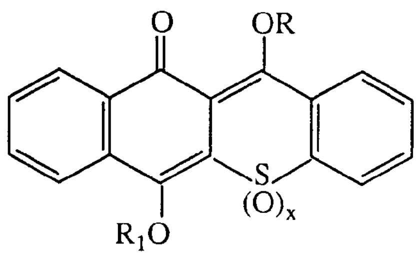

(III),

worin R, $R_1$, und x die zuvor angegebenen Bedeutungen haben, einschliesslich der Bevorzugungen.

Die Verbindungen der Formel III können nach der Bestrahlung von Lösungen der Verbindungen der Formel I durch Entfernen des Lösungsmittels erhalten und gegebenenfalls nach üblichen Methoden gereinigt werden.

Die Farbänderung ist reversibel. Bei erneuter Bestrahlung mit Licht einer Wellenlänge von etwa 450 bis 550 nm erhält man wieder die ursprüngliche Farbe. Besonders vorteilhaft ist, dass dieser Vorgang mehrfach wiederholt werden kann. Die Stabilität der photochemischen Hin- und Rückreaktion ist überraschend hoch und die Ermüdung selbst an Luft oder in Substraten entsprechend gering. So werden bei mehr als 20 Zyklen praktisch keine Veränderungen beobachtet. Als vorteilhaft ist auch anzusehen, dass die zur photochemischen Umwandlung benötigte Lichtabsorption im Bereich der Wellenlänge handelsüblicher Laser liegt.

Ein weiterer Gegenstand der Erfindung ist die Verwendung von Verbindungen der Formeln I oder III als reversible photochrome Systeme zur Kontrastbildung oder Lichtabsorption.

Die Verbindungen der Formel I können als Photoinitiatoren und besonders Photosensibilisatoren in photopolymerisierbaren Systemen verwendet werden, wobei sie gleichzeitig als Farbindikatoren wirken. So ist es möglich, belichtete Produkte (z.B. Schutzschichten, Druckplatten, Offsetdruckplatten, gedruckte Schaltungen, Lötstoppmasken) zu markieren und von unbelichteten Produkten zu unterscheiden, und ferner bei einer Produktekontrolle fehlerhaft belichtete Produkte vor oder nach der Entwicklung auszusondern.

Der erhebliche Vorteil bei der Verwendung als Farbindikatoren liegt in der Erhöhung der Sensibilisatorwirkung. Ueblicherweise als Farbumschlagsysteme eingesetzte Zusätze bewirken im allgemeinen eine Erniedrigung der Photosensibilität.

Die Verbindungen der Formeln I oder III können auch als solche, in Lösung oder in Polymeren eingearbeitet, als Photofarbindikatoren oder als Photoschaltelemente verwendet werden.

Die Verbindungen der Formel I können auch in organischen oder anorganischen Gläsern als photoschaltbare Farbfilter verwendet werden, z.B. in Glasern für Sonnenbrillen, Kontaktlinsen, Fenstern und Spiegeln.

Ein weiterer Gegenstand der Erfindung ist eine strahlungsempfindliche Zusammensetzung, enthaltend

a) ein strahlungsempfindliches organisches Material, und

b) mindestens eine Verbindung der Formeln I oder III oder Mischungen davon.

Die Verbindungen der Formel I und III oder Mischungen davon können in einer Menge von 0,001 bis 20 Gew.-%, besonders 0,001 bis 10 Gew.-% und insbesondere 0,01 bis 5 Gew.-% enthalten sein, bezogen auf die Komponente a).

Strahlungsempfindliche und damit auch photostrukturierbare Materialien sind bekannt. Es kann sich um Positiv- oder Negativsysteme handeln. Solche Materialien sind z.B. von G. E. Green et al. in J. Macromol. Sci.; Revs. Macromol. und Chem., C21(2), 187-273 (1981 bis 1982) und von G.A. Delzenne in Adv. Photochem., 11, S. 1-103 (1979) beschrieben worden.

Vorzugsweise handelt es sich bei dem strahlunbempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide.

Photopolymerisierbare Substanzen sind z.B. Acryl- und besonders Methacrylsäureester von Polyolen, z.B. Ethylenglykol, Propandiol, Butandiol, Hexandiol, Di(hydroxymethyl)-cyclohexan, Polyoxyalkylendiole wie z.B. Di-, Tri- oder Tetraethylenglykol, Di- oder Tri- 1,2-propylenglykol, Trimethylolmethan, -ethan oder -propan und Pentaerythrit, die alleine, in Mischungen und in Abmischung mit Bindemitteln verwendet werden können.

Photodimerisierbare Substanzen sind z.B. Homo- und Copolymere, die Zimtsäuregruppen oder substituierte Maleinimidylverbindungen in Seitengruppen oder Chalkongruppen in der Polymerkette enthalten.

Bevorzugt sind solche Zusammensetzungen, worin Komponente a1) ein Homo- oder Copolymer von Acryl-, Methacryl- oder Maleinsäureestern ist, deren Estergruppen einen Rest der Formel

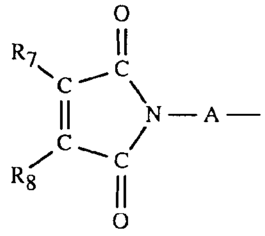

enthalten, worin A für unsubstituiertes oder mit Hydroxyl substituiertes lineares oder verzweigtes $C_2$-$C_{12}$-Alkylen, Cyclohexylen oder Phenylen steht, und $R_7$ und $R_8$ unabhängig voneinander Cl, Br, Phenyl oder $C_1$-$C_4$-Alkyl bedeuten, oder $R_7$ und $R_8$ zusammen Trimethylen, Tetramethylen oder

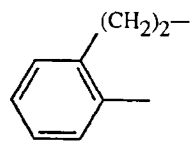

bedeuten. Solche Polymere sind z.B. in der US-A-4 193 927 beschrieben.

Die photopolymerisierbaren oder photodimerisierbaren Substanzen können weitere für die Verarbeitung oder Anwendung übliche Additive enthalten, sowie zusätzlich andere Photoinitiatoren oder Photosensibilisatoren.

Bei den kationisch härtbaren Systemen handelt es sich bevorzugt um Epoxidverbindungen mit mindestens 2 Epoxidgruppen im Molekül, denen ein Photoinitiator einverleibt ist. Geeignete Photoinitiatoren sind z.B. die bekannten Cyclopentadienyl-Aren-Metallsalze, Cyclopentadienyl-metallcarbonyl-salze und Oniumsalze. Die härtbaren Systeme können für die Verarbeitung und Anwendung übliche Zusatzstoffe enthalten.

Photoempfindliche Polyimide sind z.B. in der DE-A- 1 962 588, EP-A-0 132 221, EP-A- 0 134 752, EP-A-0 162 017, EP-A-0 181 37 und EP-A-0 182 745 beschrieben.

Die erfindungsgemässe Zusammensetzung wird nach bekannten Methoden als Schicht auf Substrate aufgebracht und entweder durch flächige Bestrahlung eine Schutzschicht oder durch Bestrahlung unter einer Photomaske oder durch ortsaufgelöste Bestrahlung mittels eines geführten Laserstrahls oder durch holographische Verfahren und nachfolgende Entwicklung ein Reliefbild erzeugt.

Ein weiterer Gegenstand der Erfindung ist eine Zusammensetzung, enthaltend

a) ein farbloses organisches Lösungsmittel, ein Polymer oder ein organisches Glas oder ein Verbundglas, und

b) gelöst, eingemischt oder als Schicht auf mindestens einer Obefläche eine Verbindung der Formel I oder III oder Mischungen davon. Die Komponente b) ist bevorzugt in einer Menge von 0,001 bis 20 Gew.-%, besonders 0,001 bis 10 Gew.-% und insbesondere 0,01 bis 5 Gew.-% enthalten, bezogen auf Komponente a). Organische Lösungen können zur Beschichtung von anderen Substanzen verwendet werden, z.B. festen Substraten wie zum Beispiel anorganischen Gläsern, die dann als photoschaltbare Substrate verwendet werden können. Die Verbindungen der Formel I oder III können auch auf Substrate aufsublimiert werden. Die beschichteten Substrate können mit einer Schutzschicht aus z.B. transparenten Polymeren versehen werden. Feste Substrate können auch mit Zusammensetzungen beschichtet werden, die ein Polymer und mindstens eine Verbindung der Formeln I oder III enthalten. Geeignete Lösungsmittel sind z.B. Kohlenwasserstoffe, halogenierte Kohlenwasserstoffe, Ketone, Carbonsäureester und Lactone, N-alkylierte Säureamide und Lactame, Alkanole und Ether.

Geeignete Polymere sind z.B. Duroplaste, Thermoplaste und strukturell vernetzte Polymere. Die Polymeren sind bevorzubt transparent. Solche Polymere und organische Gläser sind dem Fachmann geläufig. Die Einarbeitung der erfindungsgemässen Verbindungen erfolgt nach üblichen Methoden, z.B. mit Lösungsverfahren und Entfernen des Lösungsmittels, Kalandrieren oder Extrusion. Die erfindungsgemässen Verbindungen können den Substraten auch vor, während oder nach deren Herstellung einverleibt werden.

Ein weiterer Gegenstand der Erfindung ist ein Verfahren zur Herstellung von gefärbten Materialien unter Einwirkung von Licht, dadurch gekennzeichnet, dass man dem Material eine Verbindung der Formel I oder III einverleibt und darauf das Material mit Licht bestrahlt.

Ferner ist ein Gegenstand der Erfindung die Verwendung von Verbindungen der Formel I als Photosensibilisatoren und Farbindikatoren oder photoschaltbare Farbfilter bei Lichteinwirkung.

Ein weiterer Gegenstand der Erfindung ist die Verwendung der Verbindung der Formel I oder III zur reversiblen optischen Speicherung von Information, wobei die Information in einer die Verbindung enthaltenden, speicheraktiven Schicht mit Licht, bevorzugt Laserlicht, eingeschrieben wird. Die eingeschriebene Information kann bevorzugt mit Laserlicht, wieder gelöscht werden, so dass ein zyklisches Einschreiben und Löschen möglich ist.

Zur Herstellung einer speicheraktiven Schicht kann die Verbindung I oder III nach zuvor beschriebenen Verfahren in einer transparenten Matrix gelöst und in dünner Schicht auf ein ebenes Substrat aufgebracht werden. Die Dicke der speicheraktiven Schicht beträgt im allgemeinen ca. 0,1-100 μm, bevorzubt 0,3-3 μm.

Das Einschreiben der Information kann durch gerasterte, holographische oder photographische Belichtung der speicheraktiven Schicht mit spektralem, bevorzugt kohärentem (Laser-)Licht im Wellenlängenbereich 400-500 nm, bevorzugt 420-460 nm erfolgen.

Das Auslesen kann mit reduzierter Strahlunbsleistung bei der Einschreibwellenlänge oder einer anderen Wellenlänge oberhalb etwa 420 nm über die örtlich geänderte Transmission, Reflexion, Brechung oder Fluoreszenz der speicheraktiven Schicht erfolgen.

Das Löschen kann durch punktförmige oder flächige Belichtung der speicheraktiven, die Verbindungen der Formeln I und /oder III enthaltenden Schicht im Wellenbereich 250-400 nm, bevorzugt 350-400 nm erfolgen.

Ein Vorteil der erfindungsgemässen Verwendung ist, dass die zum Einschreiben, Auslesen und Löschen erforderlichen Wellenlängen im Bereich handelsüblicher Laser liegen (z.B. Argonionenlaser. 454/458 nm bzw. 351/363 nm; HeCd-Laser: 325 und 442 nm; insbesondere frequenzverdoppelte Diodenlaser mit etwa 420-430 nm beziehungsweise etwa 370-390 nm).

Ein weiterer Vorteil ist der hohe erreichbare Kontrast der Absorption zwischen dem beschriebenen und dem gelöschten Zustand im Bereich 400-500 nm und die damit verbundene grosse Dynamik der speicheraktiven Schicht.

Ein anderer Vorteil ist, dass die Quantenausbeute beim Einschreiben relativ niedrig ist und damit die Gefahr eines Ueberschreibens beim Auslesen stark reduziert wird.

Vorteilhaft ist auch umgekehrt, dass die Quantenausbeute beim Löschvorgang relativ hoch ist und damit ein rasches grossflächiges Löschen ermöglicht wird.

Ein weiterer Vorteil ist die hohe Photostabilität der Verbindung der Formel I oder III und die dadurch erreichbare hohe Zahl von Schreib-/Löschzyklen.

Schliesslich besteht als weiterer Vorteil die Möglichkeit einer zyklischen Datenauffrischung durch Zumischen eines geeigneten Quantums Licht der Löschwellenlänge während des Auslesens.

Die nachfolgenden Beispiele erläutern die Erfindung. Prozente sind Molprozente (Ausbeuten) oder Volumenprozente (Laufmittel), wenn es nicht anders angegeben ist.

A) <u>Herstellung von Ausgangverbindungen</u>

<u>Beispiel A1:</u> 6,7-Benzo-5-methoxy-8-phenoxy-thioxanthon.

a) 1,4-Dimethoxy-3-(1′-carbomethoxy-2′-phenylthio)-naphthalin.

6 g (19,2 mmol) 1,4-Dihydroxy-3-(1′-carboxyl-2′-phenylthio)-naphthalin, 13,6 g (96 mmol) Methyliodid, 13,27 g (96 mmol) Kaliumcarbonat und 60 ml Dimethylformamid werden 20 Minuten bei 25 °C gerührt. Dann wird das Gemisch in wässriger Salzsäure/Toluol aufgenommen und die organische Phase abgetrennt, zweimal mit Wasser gewaschen, über Natriumsulfat getrocknet und danach einbedampft. Nach dem Verrühren mit Methanol werden 5,05 g (74 %) kristallines Produkt mit einem Schmelzpunkt (Smp.) von 123-125 °C isoliert.

b) 1,4-Dimethoxy-3-(1′-carboxyl-2′-phenylthio)-naphthalin.

85 g (240 mmol) der Verbindung gemäss a), 40,37 g (720 mmol) KOH und 700 ml Ethanol werden 15 Minuten bei 80 °C gerührt. Nach dem Abkühlen wird das Reaktionsgemisch in verdünnte Salzsäure gegossen, die Suspension mit Tetrahydrofuran/Toluol extrahiert, die abgetrennten organischen Phasen über Natriumsulfat getrocknet und dann eingedampft. Nach der Umkristallisation aus Toluol erhält man 76,9 g (94 %) kristallines Produkt mit einem Smp. von 185-187 °C.

c) 6,7-Benzo-5-methoxy-8-chlor-thioxanthon.

30 g (88,1 mmol) der Verbindung gemäss b) werden in 400 ml o-Dichlorbenzol und 40 ml Phosphoroxytrichlorid während 6 Stunden unter Rückfluss gerührt. Das Reaktionsgemisch wird in eine Eis/Wasser-Mischung gegossen, gerührt und dann mit Tetrahydrofuran/Ethanol (1:1) extrahiert. Die abgetrennten organischen Phasen werden mit wässriger Sodalösung gewaschen, über Natriumsulfat getrocknet und dann eingedampft. Das Rohprodukt wird mit Methylenchlorid über Kieselgel filtriert und das Filtrat eingedampft. Der Rückstand wird mit Pentan verrührt und filtriert. Man erhält 23,2 g (81 %) eines gelben kristallinen Produkts, Smp.: 162-164 °C.

d) 3 g (9,18 mmol) der Verbindung gemäss c), 1,12 g (11,93 mmol) Phenol, 2,54 g ( 18,36 mmol) Kaliumcarbonat und 30 ml Dimethylsulfoxid werden 2,5 Stunden bei 120 °C gerührt. Nach dem Abkühlen wird das Reaktionsgemisch in einem Gemisch aus Tetrahydrofuran/Toluol/2N wässrige HCl aufgenommen, die organische Phase abgetrennt, über Natriumsulfat getrocknet und eingedampft. Das Rohprodukt wird in Methylenchlorid über Kieselgel filtriert und dann das Lösungsmittel verdampft. Man erhält 2,1 g (60 %) der Titelverbindung als gelbe Kristalle, Smp.: 235-238 °C.
Eine Toluollösung weist bei der Bestrahlung einen irreversiblen Farbumschlag von schwach gelb nach schwach grün auf.

Beispiel A2: 6,7-Benzo-5-methoxy-8-chlor-thioxanthonsulfodioxid.

3 g (9,18 mmol) der Verbindung gemäss Alc), 6 ml 30% $H_2O_2/H_2O$ und 30 ml Eisessig werden 2 Stunden bei 90 °C gerührt. Das Gemisch wird auf 200 ml Wasser ausgetragen; die Kristalle werden abfiltriert und mit Wasser gewaschen. Nach dem Trocknen und Umkristallisieren aus Toluol werden 2,9 g (88%) reines Produkt erhalten, Smp.: 160-162°C.

B) Herstellungsbeispiele

Beispiel B1: 6,7-Benzo-5-methoxy-8-phenoxy-thioxanthonsulfoxid.

0,5 g (1,3 mmol) Verbindung A1, 2 ml wässrige Wasserstoffperoxidlösung (30 %) und 50 ml Eisessig werden 2 Stunden bei 25 °C gerührt. Dann wird eine Mischung von Tetrahydrofuran/Toluol (1:1) zugegeben, die organische Phase abgetrennt, mit Wasser gewaschen, über Natriumsulfat getrocknet und eingedampft. Danach wird über Kieselgel chromatographiert (Laufmittel 3 % Aceton in Methylenchlorid). Man erhält 0,23 g (44 %) der Titelverbindung als gelbe Kristalle, Smp.: 230-233 °C, Massenspektrum: 400 ($M^+$). Eine Lösung der Verbindung in Toluol weist bei Bestrahlung einen reversiblen Farbumschlag von farblos nach gelb auf.

Beispiel B2: 6,7-Benzo-5-methoxy-8-phenoxy-thioxanthonsuldioxid.

0,5 g (1,3 mmol) Verbindung A1, 1 ml wässrige Wasserstoffperoxidlösung (30 %) und 10 ml Eisessig werden 3 Stunden bei 80 °C gerührt. Dann wird eine Mischung von Tetrahydrofuran/Toluol (1:1) zugegeben, die organische Phase abgetrennt, mit Wasser gewaschen, über Natriumsulfat getrocknet und eingedampft. Danach wird über Kieselgel chromatographiert (Laufmittel 3 % Aceton in Methylenchlorid). Man erhält 0,46 g (44 %) der Titelverbindung als gelbe Kristalle, Smp.: 210-213 °C, Massenspektrum: 416 ($M^+$).
Eine Lösung der Verbindung in Toluol weist bei Bestrahlung einen reversiblen Farbumschlag von farblos nach gelb auf.

Beispiel B3: 6,7-Benzo-5-benzyloxy-8-phenoxy-thioxanthonsulfodioxid.

a) 5-Hydroxy-8-phenyloxy-thioxanthonsulfodioxid.

3 g (7,2 mmol) Verbindung B2 und 30 ml HBr-Lösung in Eisessig (30 %) werden 1 Tag bei 130 °C im Autoklaven gehalten. Nach dem Abkühlen wird das Gemisch in Wasser gegossen, der Niederschlag abfiltriert und dann in Tetrahydrofuran/Toluol (1: 1) gelöst. Die Lösung wird mit 2N NaOH gewaschen, über Natriumsulfat getrocknet und dann eingedampft. Danach wird der Rückstand in Methylenchlorid aufgenommen und die Lösung über Kieselgel filtriert. Man erhält 1,2 g (42 %) Produkt als orange Kristalle.

b) 1 g (2,48 mmol) dieses Produkts, 2,13 g (12,43 mmol) Benzylbromid, 2,06 g (14,91 mmol) Kaliumcarbonat und 10 ml Dimethylformamid werden 25 Minuten bei 50 °C gerührt und das Reaktionsgemisch in eine Mischung aus Tetrahydrofuran/Toluol/2N HCl gegossen. Die organische Phase wird abgetrennt, mit Natriumsulfat getrocknet und über basischem Aluminiumoxid filtriert. Durch Umkristallisation aus Methylenchlorid/Pentan (1:1) erhält man 1 g (82 %) der Titelverbindung, Smp.: 155-160 °C, Massenspektrum: 492 ($M^+$). Eine Lösung der Verbindung in Toluol weist bei Bestrahlung einen reversiblen Farbumschlag von farblos nach gelb auf.

Beispiel B4: 6,7-Benzo-5-butoxy-8-phenoxy-thioxanthonsulfodioxid.

a) 6,7-Benzo-5-hydroxy-8-brom-thioxanthonsulfodioxid.

2,08 g (5mmol) Verbindung B2 in 50 ml Methylenchlorid werden bei 5 °C mit 0,35 ml $BBr_3$ versetzt, auf 25 °C erwärmt und 10 Minuten gerührt. Dann wird langsam Wasser zugetropft und danach mit Tetrahydrofuran/Toluol (1:1) extrahiert. Die organische Phase wird mit Wasser gewaschen, über Natriumsulfat getrocknet und eingedampft. Der Rückstand wird in Toluol gelöst, heiss filtriert und dann kristallisiert. Man erhält 1,75 g (90 %) der Verbindung, Massenspektrum: 388/390 ($M^+$).

b) 6,7-Benzo-5-butoxy-8-brom-thioxanthonsulfodioxid.

0,53 g (1,36 mmol) der Verbindung gemäss a), 0,56 g (4,09 mmol) Kaliumcarbonat, 0,38 g (2,04 mmol) Butyliodid und 10 ml N-Methylpyrrolidon werden 18 Stunden bei 60 °C gerührt. Das Reaktionsgemisch wird in Wasser gegossen und danach mit Tetrahydrofuran/Toluol (1:1) extrahiert.Die organische Phase wird mit Wasser gewaschen, über Natriumsulfat getrocknet und über Kieselgel filtriert. Man kristallisiert aus Methylenchlorid/Hexan um und erhält 0,3 g (50 %) der Verbindung, Smp.: 157-158 °C, Massenspektrum: 444/446 ($M^+$) und 388/390 (Basispeak).

c) 0,2 g (0,45 mmol) der Verbindung b), 0,13 g (1,35 mmol) Phenol, 0,25 g (1,8 mmol) Kaliumcarbonat und 5 ml N-Methylpyrrolidon werden 4 Stunden bei 70 °C gerührt. Das Reaktionsgemisch wird in 2N Salzsäure gegossen und mit Toluol extrahiert. Die organische Phase wird mit 2N NaOH gewaschen, dann mit Wasser gewaschen, über Natriumsulfat getrocknet und eingedampft. Man kristallisiert aus Methylenchlorid/Hexan um und erhält 158 mg (77 %) der Titelverbindung, Smp.: 172-173 °C, Massenspektrum: 458 ($M^+$) und 402 (Basispeak). Eine Lösung der Verbindung in Toluol weist bei Bestrahlung einen reversiblen Farbumschlag von farblos nach gelb auf.

Beispiel B5: 6,7-Benzo-5-methoxy-8-(3,5-dichlor-phenoxy)-thioxanthonsulfodioxid.

1 g (2,79 mmol) Verbindung A2, 0,55 g (3,34 mmol) 3,5-Dichlorphenol, 0,77 g (5,57 mmol) $K_2CO_3$ und 10 ml Dimethylsulfoxid werden während 25 Min. bei 100 °C gerührt. Das Gemisch wird in 2N HCl/Toluol aufgenommen, die organische Phase abgetrennt, mit $Na_2SO_4$ getrocknet und eingedampft. Aus Toluol umkristallisiert, werden 0,96 g (71%) reines Produkt erhalten, Smp.: 207-210 °C.

Beispiel B6: 6,7-Benzo-5-methoxy-8-(p-ethoxy-carbonyl-phenoxy)-thioxanthonsulfodioxid.

Diese Verbindung wird analog zu Beispiel B5 hergestellt, aber unter Verwendung von 4-Hydroxybenzoesäure-ethylester anstelle des 3,5-Dichlorphenols. Man erhält 0,88 g (65%) reines Produkt, Smp.: 178-180 °C.

C) Anwendungsbeispiele

Beispiel C1:

25 mg Verbindung B2 und 2,5 g Polystyrol werden bei 60 °C unter Argon und Lichtausschluss in 25 ml Toluol gelöst. Die auf Raumtemperatur erkaltete Lösung wird mit einer Rakel in 200 μm Nassfilmdicke auf einen Glasträger aufgetragen und bei 80 °C 60 Minuten in einem Umluftofen getrocknet. Man erhält einen homogenen, transparenten und freistehenden Film.

Der Film wird auf einer Quarzglasplatte im Probenraum eines Spektralphotometers montiert und mit einer 300 W Xenonlampe über Glasfasern und einen UV-Filter (Schott UG11) bestrahlt. Die integrale Bestrahlungsstärke beträgt 0,8 $mW/cm^2$. In Abständen von jeweils etwa 60 s wird die Bestrahlung unterbrochen und das Absorptionsspektrum gemessen. Das Spektrum der Probe ändert sich von farblos (optische Dichte 0,6 bei 300

nm, 0,3 bei 375 nm und null oberhalb 420 nm) nach gelb, verursacht durch eine breite Absorptionsbande im Bereich von 350 bis 500 nm (maximale optische Dichte 0,5 bei 430 nm). Die Zeitkonstante der Umwandlung beträgt 130 s.

Für die Rückreaktion wird der UV-Filter durch ein gelbes Kantenfilter (Schott GG475) mit Durchlass oberhalb etwa 400 nm ausgetauscht. Die integrale Bestrahlungsstärke im Bereich 400 bis 500 nm beträgt 2,5 mW/cm$^2$. Durch die Bestrahlung verschwindet die langwellige Absorptionsbande bei 350 bis 500 nm bis auf eine optische Dichte von maximal 0,1. Die Zeitkonstante der Rückreaktion beträgt 200 s.

Bei weiteren Belichtungszyklen bleiben die Grenzwerte der optischen Dichte konstant (bei 430 nm: etwa 0,1/0,5).

Beispiel C2:

Der Film gemäss Beispiel C1 wird zwischen zwei Quarzglasplatten in der Filmebene eines holographischen Aufzeichnungsgeräts montiert. Aus einem aufgeweiteten, raumgefilterten Argonlaserstrahl (454 nm, $\phi$ etwa 0,5 cm) werden zwei ebene Wellen (O und R) von je 2,5 mW/cm$^2$ Bestrahlungsstärke gebildet und unter einem Winkel von 3° in der Filmebene zur Koinzidenz gebracht. Aus einer 300 W Xenonlampe wird über einen UV-Filter (Schott UG11) und eine Quarzfaser ein mit O und R koinzidierendes UV-Lichtbündel (0,5 mW/cm$^2$) auf die Filmebene gerichtet. Hinter der Filmebene wird in Richtung der ersten Beugungsordnung von R, bzw. der zweiten von O, ein Detektor zur Messung des Beugungswirkungsgrades (BWG) befestigt.

Der Film wird durch zehnminütige Bestrahlung mit UV-Licht in die gelbe Form übergeführt. Nach Einschalten von O und R steigt der BWG innerhalb von 60 s monoton von 0 auf etwa 0,1 % (einschreiben). Nach Unterbrechen von O steigt der BWG sprunghaft auf etwa 0,12 % (Wegfall der destruktiv interferierenden 2. Beugungsordnung von O) und nimmt dann näherungsweise exponentiell mit einer Zeitkonstante von etwa 60 s ab (überschreiben). Durch erneute UV-Bestrahlung wird wieder gelöscht. Nach 10 Zyklen ist keine Verminderung des BWG feststellbar.

Bei gleichzeitigem Einschreiben und Löschen (holographischer Kurzzeitspeicher) wird ein stationärer BWG von etwa 0,05 % erreicht.

## Patentansprüche

1. Verbindungen der Formel I oder Mischungen solcher Verbindungen,

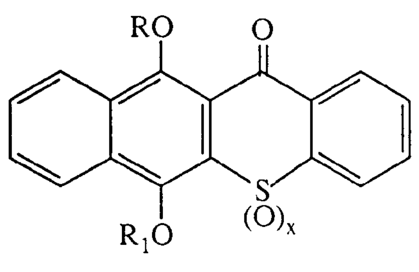

(I),

worin
R unsubstituiertes oder mit $C_1$-$C_{12}$-Alkyl, $C_1$-$C_{12}$-Alkoxy, $C_1$-$C_{12}$-Alkylthio, Phenyl, Benzyl, -CN, -$CF_3$, Halogen oder -$COOR_3$ substituiertes $C_6$-$C_{14}$-Aryl bedeutet, x für 1 oder 2 steht und $R_1$ H, lineares oder verzweigtes $C_1$-$C_{12}$-Alkyl, $C_2$-$C_{12}$-Alkenyl oder -Alkinyl, $C_7$-$C_{16}$-Aralkyl, $C_8$-$C_{16}$-Alkaralkyl, -$CH_2COOR_3$ oder $C_1$-$C_{12}$-Acyl darstellt, worin $R_3$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht.

2. Verbindungen gemäss Anspruch 1, worin R in Formel I unsubstituiertes oder substituiertes $C_6$-$C_{10}$-Aryl darstellt.

3. Verbindungen gemäss Anspruch 1, worin R in Formel I unsubstituiertes oder substituiertes Phenyl darstellt.

4. Verbindungen gemäss Anspruch 1, worin R in Formel I unsubstituiert oder mit $C_1$-$C_4$-Alkyl, $C_1$-$C_4$-Alkoxy, $C_1$-$C_4$-Alkylthio, -F, -Cl, -Br oder -$COOR_3$ substituiert ist, wobei $R_3$ H oder $C_1$-$C_{18}$-Alkyl bedeutet.

5. Verbindungen gemäss Anspruch 1, worin R in Formel I Phenyl darstellt, das unsubstituiert oder mit -C1, $-COOH_3$ oder $-COOC_2H_5$ substituiert ist.

6. Verbindungen gemäss Anspruch 1, worin $R_1$ in Formel I Alkyl mit 1 bis 8 C-Atomen, Alkenyl oder Alkinyl mit 2 bis 6 C-Atomen, Aralkyl mit 7 bis 12 C-Atomen oder Alkaralkyl mit 8 bis 12 C-Atomen darstellt.

7. Verbindungen gemäss Anspruch 1, worin $R_1$ in Formel I für $C_1$-$C_6$-Alkyl Benzyl oder ($C_1$-$C_4$-Alkyl)benzyl steht.

8. Verbindungen gemäss Anspruch 1, worin x in Formel I für 2 steht.

9. Verbindungen gemäss Anspruch 1, worin R in Formel I für Phenyl, $R_1$ für Methyl, Butyl oder Benzyl, und x für 2 stehen.

10. Verbindungen gemäss Anspruch 1, worin R in Formel I Phenyl, p-Chlorphenyl, 3,5-Dichlorphen-1-yl, p-(Carbomethoxy)phenyl oder p-(Carboethoxy)phenyl darstellt.

11. Verfahren zur Herstellung von Verbindungen der Formel I gemäss Anspruch 1, dadurch gekennzeichnet, dass man eine Verbindung der Formel II

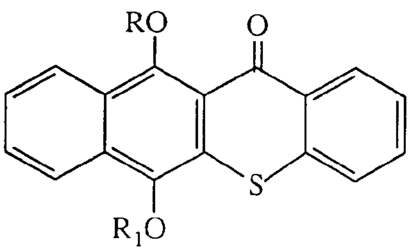

(II),

worin R und $R_1$ die in Anspruch 1 angegebenen Bedeutungen haben, mit einem Sauerstoff abgebenden Oxidationsmittel umsetzt.

12. Verbindungen der Formel II,

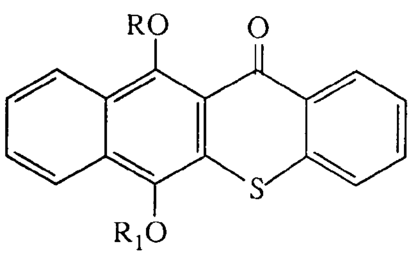

(II),

worin R und $R_1$ die in Anspruch 1 angegebenen Bedeutungen haben.

13. Verbindungen der Formel III,

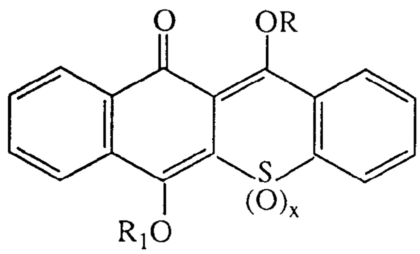

(III),

worin R, $R_1$, und x die in Anspruch 1 angegebenen Bedeutungen haben.

**14.** Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche eine Verbindung der Formel I oder III gemäss den Ansprüchen 1 oder 12 oder Mischungen davon.

**15.** Verwendung von Verbindungen der Formeln I oder III gemäss den Ansprüchen 1 oder 12 oder Mischungen davon als reversible photochrome Systeme zur Kontrastbildung oder Lichtabsorption.

**16.** Verwendung von Verbindungen der Formeln I oder III gemäss den Ansprüchen 1 oder 12 oder Mischungen davon zur reversiblen optischen Speicherung von Informationen.

Europäisches Patentamt

# EUROPÄISCHER RECHERCHENBERICHT

Nummer der Anmeldung

EP 91 81 0982

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 110 832 (CIBA-GEIGY)<br>* Seite 1 - Seite 2 *<br>--- | 1,12,14 | C07D335/04<br>G03F7/00<br>G11B7/24 |
| A | CHEMICAL ABSTRACTS, vol. 100, no. 2, 9. Januar 1984, Columbus, Ohio, US;<br>abstract no. 8573T,<br>Seite 100 ;<br>& JP-A-58 120 605 (NIPPON KAYAKU) 18. Juli 1983<br>* Zusammenfassung *<br>--- | 1,14 | |
| A<br><br><br><br><br><br><br>D | CHEMICAL ABSTRACTS, vol. 94, no. 7, 16. Februar 1981, Columbus, Ohio, US;<br>abstract no. 47012M,<br>YU.E. GERASIMENKO: 'Photochromism of peri-arylhydroxy-p-quinones. Synthesis of some derivatives of phenoxynaphthacenequinones'<br>Seite 548 ;<br>& ZHURNAL ORGANICHESKOI KHIMII, 1971, 7(11), 2413-2415<br>--- | 1,12-14 | |
| P,A | EP-A-0 430 881 (CIBA-GEIGY)<br>* Seite 2 - Seite 3; Ansprüche 1,21 *<br>----- | 1,12-14 | **RECHERCHIERTE SACHGEBIETE (Int. Cl.5 )**<br>C07D |

Der vorliegende Recherchenbericht wurde für alle Patentansprüche erstellt

| Recherchenort | Abschlußdatum der Recherche | Prüfer |
|---|---|---|
| DEN HAAG | 11 MAERZ 1992 | RUSSELL F. ENGLISH |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)